# EUROPEAN PATENT APPLICATION

(11) **EP 4 550 344 A1**
(43) Date of publication of application: **07.05.2025**
(21) Application number: 23206691.0
(22) Date of filing: 30.10.2023
(51) Int. Cl.: G16H 15/00, G16H 40/63, G16H 50/30, A61B 5/145, A61B 5/00

(54) **METHOD AND SYSTEMS FOR INTER-APPLICATION USER INTERFACE GENERATION**

(71) Applicant: Roche Diabetes Care GmbH, 68305 Mannheim (DE); F. Hoffmann-La Roche AG, 4070 Basel (CH)
(72) Inventor: KOEHLER, Matthias, 68305 Mannheim (DE); BABION, Nils, 68305 Mannheim (DE); BOS, Hans, 9711 Groningen (NL)
(74) Representative: Altmann Stößel Dick Patentanwälte PartG mbB

(57) **Abstract**

A method for generating diabetes management parameter user interfaces includes providing stored program instructions of a first diabetes management application (DMA), the first DMA configured to be stored in a memory of an electronic device and, upon execution by a processor, the first DMA is configured to generate a first output display of a plurality of diabetes management parameters corresponding to a time series of analyte data, the plurality of diabetes management parameters depicting a trend of diabetes management parameter levels over time, generate a graphical indicator of a link to a second DMA stored in the memory, and activate the second DMA in response to an input selection of the link, wherein the second DMA is configured to be executed by the processor to generate a second output display of a current-time diabetes management parameter corresponding to the time series of the analyte data stored in the memory.

## Description

### TECHNICAL FIELD

This disclosure is directed to systems and methods to coordinate the functions of multiple software applications in general and, in particular, to coordinate the functions of multiple diabetes management applications for Persons with Diabetes.

### BACKGROUND

Some Persons with Diabetes (PwDs) utilize continuous glucose monitor (CGM) devices that are affixed to or embedded in the body of the PwD and generate a time series of measurements corresponding to the blood glucose levels in the body of the PwD. Many modern CGMs transmit the blood glucose measurement data to one or more external electronic devices such as smartphones, smart watches, personal computers, and the like to enable the PwD to review the blood glucose measurement data and to enable one or more software programs, which are referred to as Diabetes Management Applications (DMAs), to access the blood glucose measurement data to provide additional analysis and treatment recommendations to the PwD.

While existing DMA software can provide sophisticated analytical and treatment advice tools to a PwD, a single DMA is often not appropriate for all situations that a PwD encounters while using the CGM. Many PwDs employ two or more DMAs where one DMA is a simplified program that provides basic information from the CGM such as the current blood glucose measurement, current time-in-range, and other short-term data from the CGM. Another DMA is a more sophisticated program that analyzes larger sets of CGM data to provide analysis and treatment recommendations. While the simplified DMA may lack every feature of the more sophisticated DMA, in many instance the PwD only requires simplified information in a summarized manner. Similarly, the PwD may need to access the more sophisticated DMA in situations where the simplified DMA does not provide sufficient functionality. However, in some instances multiple DMAs present redundant information to the PwD. Furthermore, the PwD may become confused as to which DMA provides appropriate functionality, which results in the PwD spending more time switching between DMAs to receive the desired information. Consequently, improvements to systems and methods that provide improved user interfaces and displays of diabetes management data and that improve the efficiency of the user experience during the operation of multiple DMAs would be beneficial.

### SUMMARY

In one embodiment, a method for generating output displays of diabetes management parameters has been developed. The method includes providing stored program instructions of a first diabetes management application, the first diabetes management application being configured to be stored in a memory of an electronic device and, upon execution by a processor in the electronic device. The first diabetes management application is configured to generate a first output display of a plurality of diabetes management parameters based at least in part on a time series of analyte data stored in the memory, the plurality of diabetes management parameters depicting a trend of diabetes management parameter levels over time, generate a graphical indicator of a link to a second diabetes management application stored in the memory of the electronic device in the first output display, and activate the second diabetes management application stored in the memory in response to an input selection of the link. The second diabetes management application is configured to be executed by the processor to generate a second output display of a current-time diabetes management parameter based at least in part on the time series of the analyte data stored in the memory.

The method may be computer implemented. The term "computer implemented" as used herein is a broad term and is to be given its ordinary and customary meaning to a person of ordinary skill in the art and is not to be limited to a special or customized meaning. The term specifically may refer, without limitation, to a method involving at least one computer and/or at least one computer network. The computer and/or computer network may comprise at least one processor which is configured for performing at least one of the method steps of the method according to the present invention. Preferably each of the method steps is performed by the computer and/or computer network. The method may be performed completely automatically, specifically without user interaction.

In another embodiment, an electronic device configured to generate diabetes management parameter user interfaces has been developed. The electronic device includes a memory, a display device, and a processor operatively connected to the memory and the display device. The memory is configured to store stored program instructions of a first diabetes management application and stored program instructions of a second diabetes management application. The processor is operatively connected to the memory and the display device, the processor is configured to execute the first diabetes management application and the second diabetes management application to generate a first output display of a plurality of diabetes management parameters based at least in part on a time series of analyte data stored in the memory, the plurality of diabetes management parameters depicting a trend of diabetes management parameter levels over time, generate a graphical indicator of a link to a second diabetes management application stored in the memory of the electronic device, and activate the second diabetes management application stored in the memory in response to an input selection of the link to generate a second output display of a current-time diabetes management parameter based at least in part on the time series of the analyte data stored in the memory.

Further disclosed and proposed herein is a computer program including computer-executable instructions for performing the method according to the present invention in one or more of the embodiments enclosed herein when the instructions are executed on a computer or computer network. Specifically, the computer program may be stored on a computer-readable data carrier and/or on a computer-readable storage medium.

As used herein, the terms "computer-readable data carrier" and "computer-readable storage medium" specifically may refer to non-transitory data storage means, such as a hardware storage medium having stored thereon computer-executable instructions. The computer-readable data carrier or storage medium specifically may be or may comprise a storage medium such as a random-access memory (RAM) and/or a read-only memory (ROM).

Thus, specifically, one, more than one or even all of method steps as indicated above may be performed by using a computer or a computer network, preferably by using a computer program.

Further disclosed and proposed herein is a computer program product having program code means, in order to perform the method according to the present invention in one or more of the embodiments enclosed herein when the program is executed on a computer or computer network. Specifically, the program code means may be stored on a computer-readable data carrier and/or on a computer-readable storage medium.

Further disclosed and proposed herein is a data carrier having a data structure stored thereon, which, after loading into a computer or computer network, such as into a working memory or main memory of the computer or computer network, may execute the method according to one or more of the embodiments disclosed herein.

Further disclosed and proposed herein is a non-transient computer-readable medium including instructions that, when executed by one or more processors, cause the one or more processors to perform the method according to one or more of the embodiments disclosed herein.

Further disclosed and proposed herein is a computer program product with program code means stored on a machine-readable carrier, in order to perform the method according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier and/or on a computer-readable storage medium. Specifically, the computer program product may be distributed over a data network.

Finally, disclosed and proposed herein is a modulated data signal which contains instructions readable by a computer system or computer network, for performing the method according to one or more of the embodiments disclosed herein.

Referring to the computer-implemented aspects of the invention, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

Specifically, further disclosed herein are:
- a computer or computer network comprising at least one processor, wherein the processor is adapted to perform the method according to one of the embodiments described in this description,
- a computer loadable data structure that is adapted to perform the method according to one of the embodiments described in this description while the data structure is being executed on a computer,
- a computer program, wherein the computer program is adapted to perform the method according to one of the embodiments described in this description while the program is being executed on a computer,
- a computer program comprising program means for performing the method according to one of the embodiments described in this description while the computer program is being executed on a computer or on a computer network,
- a computer program comprising program means according to the preceding embodiment, wherein the program means are stored on a storage medium readable to a computer,
- a storage medium, wherein a data structure is stored on the storage medium and wherein the data structure is adapted to perform the method according to one of the embodiments described in this description after having been loaded into a main and/or working storage of a computer or of a computer network, and
- a computer program product having program code means, wherein the program code means can be stored or are stored on a storage medium, for performing the method according to one of the embodiments described in this description, if the program code means are executed on a computer or on a computer network.

Summarizing and without excluding further possible embodiments, the following embodiments may be envisaged:
Embodiment 1. A method for generating output displays of diabetes management parameters comprising:
   - providing stored program instructions of a first diabetes management application, the first diabetes management application being configured to be stored in a memory of an electronic device and, upon execution by a processor in the electronic device, the first diabetes management application being configured to:
      generate a first output display of a plurality of diabetes management parameters based at least in part on a time series of analyte data stored in the memory, the plurality of diabetes management parameters depicting a trend of diabetes management parameter levels over time;
      generate a graphical indicator of a link to a second diabetes management application stored in the memory of the electronic device in the first output display; and
      activate the second diabetes management application stored in the memory in response to an input selection of the link, wherein the second diabetes management application is configured to be executed by the processor to generate a second output display of a current-time diabetes management parameter based at least in part on the time series of the analyte data stored in the memory.
Embodiment 2. The method of embodiment 1, wherein the plurality of diabetes management parameters are glucose management indicators (GMIs) and the current-time diabetes management parameter is a current-time GMI.
Embodiment 3. The method of embodiment 2, wherein upon execution by the processor, the first diabetes management application is further configured to:
   generate the first output display including a first graphical indicator of an average GMI occurring in a prior time range and a second graphical indicator of an average GMI occurring in a present time range.
Embodiment 4. The method of embodiment 1, wherein the plurality of diabetes management parameters are Time in Range (TIR) parameters and the current-time diabetes management parameter is a current-time TIR parameter.
Embodiment 5. The method of embodiment 4, wherein upon execution by the processor, the first diabetes management application is further configured to:
   generate the first output display including a series of at least two bars and a trend line, wherein each bar includes color indications of TIR during a time period corresponding to the bar and the trend line indicates changes in TIR over time between the at least two bars.
Embodiment 6. The method of embodiment 1, wherein the plurality of diabetes management parameters are a trend of predicted analyte levels and the current-time diabetes management parameter is a current-time analyte level.
Embodiment 7. The method of embodiment 6, wherein upon execution by the processor, the first diabetes management application is further configured to:
   generate the first output display including a linear graph of a prediction of future analyte values.
Embodiment 8. The method of embodiment 1 further comprising:
   providing the stored program instructions of the second diabetes management application, the second diabetes management application being configured to be stored in the memory of the electronic device and to be executed by the processor in the electronic device.
Embodiment 9. The method of embodiment 1 wherein upon execution by the processor, the first diabetes management application is further configured to:
   receive the time series of analyte measurement data from an analyte sensor, the time series of analyte data being accessible to the first diabetes management application and
   the second diabetes management application.
Embodiment 10. The method of embodiment 1 wherein the time series of analyte measurement data is a time series of blood glucose measurement data.
Embodiment 11. An electronic device configured to generate diabetes management parameter user interfaces comprising:
   a memory configured to store:
      stored program instructions of a first diabetes management application; and
      stored program instructions of a second diabetes management application;
   a display device; and
   a processor operatively connected to the memory and the display device, the processor configured to execute the first diabetes management application and the second diabetes management application to:
      generate a first output display of a plurality of diabetes management parameters based at least in part on a time series of analyte data stored in the memory, the plurality of diabetes management parameters depicting a trend of diabetes management parameter levels over time;
      generate a graphical indicator of a link to a second diabetes management application stored in the memory of the electronic device; and
      activate the second diabetes management application stored in the memory in response to an input selection of the link to generate a second output display of a current-time diabetes management parameter based at least in part on the time series of the analyte data stored in the memory.
Embodiment 12. The electronic device of embodiment 11, wherein the plurality of diabetes management parameters are glucose management indicators (GMIs) and the current-time diabetes management parameter is a current-time GMI.
Embodiment 13. The electronic device of embodiment 12, wherein the processor is further configured to:
   execute the first diabetes management application to generate the first output display including a first graphical indicator of an average GMI occurring in a prior time range and a second graphical indicator of an average GMI occurring in a present time range.
Embodiment 14. The electronic device according to any one of the preceding embodiments referring to an electronic device, wherein the plurality of diabetes management parameters are Time in Range (TIR) parameters and the current-time diabetes management parameter is a current-time TIR parameter.
Embodiment 15. The electronic device of embodiment 14, wherein the processor is further configured to:
   execute the first diabetes management application to generate the first output display including a series of at least two bars and a trend line, wherein each bar includes color indications of TIR during a time period corresponding to the bar and the trend line indicates changes in TIR over time between the at least two bars.
Embodiment 16. The electronic device according to any one of the preceding embodiments referring to an electronic device, wherein the plurality of diabetes management parameters are a trend of predicted analyte levels and the current-time diabetes management parameter is a current-time analyte level.
Embodiment 17. The electronic device of embodiment 16, wherein the processor is further configured to:
   execute the first diabetes management application to generate the first output display including a linear graph of a prediction of future analyte values.
Embodiment 18. The electronic device of according to any one of the preceding embodiments referring to an electronic device, wherein the processor is further configured to:
   receive the time series of analyte measurement data from an analyte sensor using a communication interface, the time series of analyte data being accessible to the first diabetes management application and the second diabetes management application.
Embodiment 19. The electronic device of according to any one of the preceding embodiments referring to an electronic device wherein the time series of analyte measurement data is a time series of blood glucose measurement data.

### BRIEF DESCRIPTION OF THE SEVERAL VIEWS OF THE DRAWINGS

To easily identify the discussion of any particular element or act, the most significant digit or digits in a reference number refer to the figure number in which that element is first introduced.
FIG. 1 is a schematic diagram of a system that includes an analyte sensor, and electronic device that executes multiple Diabetes Management Applications (DMAs).
FIG. 2 is a block diagram of a method for operating multiple DMAs.
FIG. 3A is a depiction of an output display that provides a long term GMI trend data display in a first DMA.
FIG. 3B is a depiction of an output display of a current-time Glucose Management Indicator (GMI) display a second DMA.
FIG. 4A is a depiction of an output display that provides a long term TIR trend data display in a first DMA.
FIG. 4B is a depiction of an output display that provides a current Time In Range (TIR) display in a second DMA.
FIG 5A is a depiction of an output display that provides a predicted trend of future blood glucose levels in a first DMA.
FIG. 5B is a depiction of an output display provides a current blood glucose level display in a second DMA.

### DETAILED DESCRIPTION

These and other advantages, effects, features and objects are better understood from the following description. In the description, reference is made to the accompanying drawings, which form a part hereof and in which there is shown by way of illustration, not limitation, embodiments of the inventive concept. Corresponding reference numbers indicate corresponding parts throughout the several views of the drawings.

While the inventive concept is susceptible to various modifications and alternative forms, exemplary embodiments thereof are shown by way of example in the drawings and are herein described in detail. It should be understood, however, that the description of exemplary embodiments that follows is not intended to limit the inventive concept to the particular forms disclosed, but on the contrary, the intention is to cover all advantages, effects, and features falling within the spirit and scope thereof as defined by the embodiments described herein and the embodiments below. Reference should therefore be made to the embodiments described herein and embodiments below for interpreting the scope of the inventive concept. As such, it should be noted that the embodiments described herein may have advantages, effects, and features useful in solving other problems.

The devices, systems and methods now will be described more fully hereinafter with reference to the accompanying drawings, in which some, but not all embodiments of the inventive concept are shown. Indeed, the devices, systems and methods may be embodied in many different forms and should not be construed as limited to the embodiments set forth herein; rather, these embodiments are provided so that this disclosure will satisfy applicable legal requirements.

Likewise, many modifications and other embodiments of the devices, systems and methods described herein will come to mind to one of skill in the art to which the disclosure pertains having the benefit of the teachings presented in the foregoing descriptions and the associated drawings. Therefore, it is to be understood that the devices, systems and methods are not to be limited to the specific embodiments disclosed and that modifications and other embodiments are intended to be included within the scope of the embodiments. Although specific terms are employed herein, they are used in a generic and descriptive sense only and not for purposes of limitation.

Unless defined otherwise, all technical and scientific terms used herein have the same meaning as commonly understood by one of skill in the art to which the disclosure pertains. Although any methods and materials similar to or equivalent to those described herein can be used in the practice or testing of the methods, the preferred methods and materials are described herein.

Moreover, reference to an element by the indefinite article "a" or "an" does not exclude the possibility that more than one element is present, unless the context clearly requires that there be one and only one element. The indefinite article "a" or "an" thus usually means "at least one." Likewise, the terms "have," "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. For example, the expressions "A has B," "A comprises B" and "A includes B" may refer both to a situation in which, besides B, no other element is present in A (*i.e.,* a situation in which A solely and exclusively consists of B) or to a situation in which, besides B, one or more further elements are present in A, such as element C, elements C and D, or even further elements.

As used herein, the term Person with Diabetes (PwD) refers to a patient who is diagnosed with or is at-risk for being diagnosed with one or more forms of diabetes including pre-diabetes, type 1 diabetes, type 2 diabetes, gestational diabetes, as well as one or more comorbidities that are associated with diabetes.

As used herein, the term "diabetes management parameter" refers to any quantifiable aspect of a PwD's physiology that is either identified directly or calculated from one or more measurements, such as blood glucose or other physiological measurements, as part of providing diabetes management services to a PwD. A non-limiting list of diabetes management parameters that are of interest to the treatment of diabetes and diabetes comorbidities includes blood glucose levels, glucose management index (GMI) values, time in range (TIR) values, glycosylated hemoglobin (HbA1c), blood ketones, estimated glomerular filtration rate (eGFR), and blood pressure (BP).

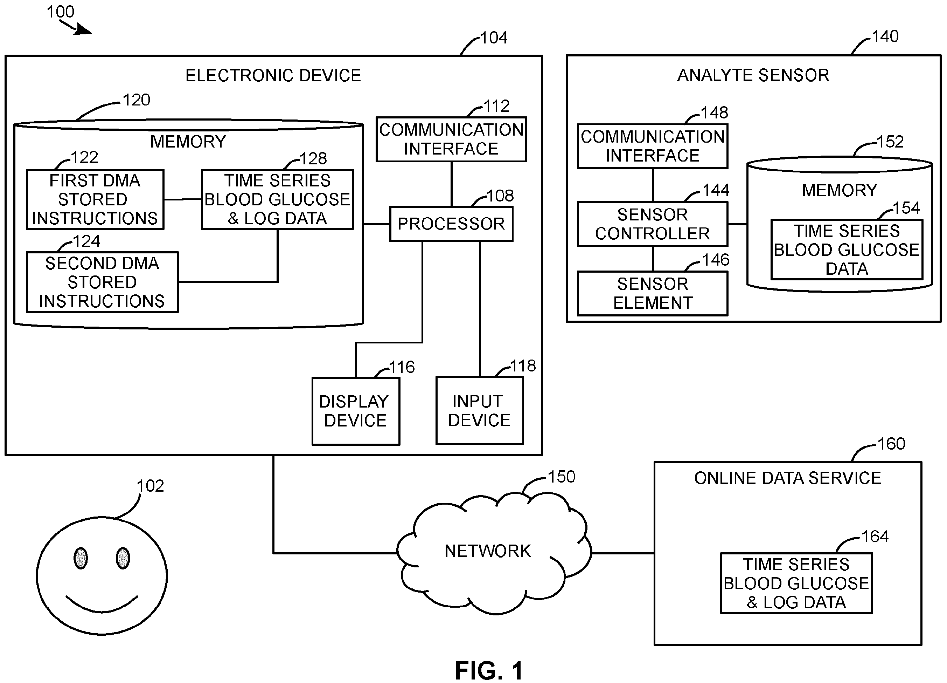

Fig. 1 depicts a diabetes management system 100 that includes an electronic device 104, an analyte sensor 140, and an optional online data service 160. In FIG. 1, a user 102 is a PwD who uses the diabetes management system 100 to manage his or her diabetes, including using the diabetes management system 100 to operate the analyte sensor 140 and to receive analyte data, such as blood glucose measurement data, with the electronic device 104. As described in further detail below, the electronic device 104 executes multiple diabetes management applications (DMAs) that process the analyte data to provide analysis and treatment recommendations to the PwD 102.

In the system 100, the electronic device 104 includes an electronic device processor 108 that is operatively connected to a communication interface 112, input/output (I/O) devices 116, and a memory 120. While the specific attributes and operations of certain components in the electronic device 104 that are relevant to detecting and recording meal events and generating medication bolus recommendations are described in further detail herein, in at least some embodiments the electronic device 104 is embodied as a commercially available smartphone, tablet computer, personal computer, a wearable device such as a smartwatch or fitness tracker, or another suitable electronic device that the user 102 operates.

In the electronic device 104, the processor 108 is a digital logic device formed from one or more integrated circuits that typically includes one or more central processing unit (CPU) cores and a graphical processing unit (GPU) to execute stored program instructions that implement an operating system and application software and to provide a graphical user interface via one or more display devices, such as the display device 116 in FIG. 1. The processor 108 further incorporates peripheral device and memory controllers that provide operative connections to the communication interface 112, one or more input devices 118, and the memory 120. Amongst other software programs, the processor 108 executes stored instructions for a first Diabetes Management Application (DMA) 122 and a second DMA 124 that are stored in the memory 120. The processor 108 also receives blood glucose data or other analyte data from the analyte sensor 140 via the communication interface 112 and stores the blood glucose data as time series blood glucose data 128 in the memory 120. The memory 120 also stores log book entries, which include records of diabetes events such as hyperglycemic episodes, hypoglycemic episodes, meal times, and medication administration events such as insulin injections or the administration of other medications such as Metformin as log book entries 128. Each log book entry is associated with one or more blood glucose levels measured at or around the time of the diabetes event. Additional elements of the processor 108 optionally include digital signal processors (DSPs), image processing units, neural network accelerators, analog-to-digital converters, digital-to-analog converters, and any other suitable digital and analog electronic components. FIG. 1 depicts the processor 108, communication interface 112, and memory 120 as separate components for explanatory purposes, but in some embodiments the processor 108 is implemented with a System on a Chip (SoC) configuration that incorporates some or all of these components into a single device.

In the electronic device 104, the communication interface 112 is, for example, a wireless or wired peripheral interface that provides a direct communication channel between the electronic device 104 and the analyte sensor 140. Non-limiting examples of wireless communication interface devices include a Bluetooth transceiver that implements one or more of the Bluetooth family of protocols including Bluetooth low-energy (BLE) and a near field communication (NFC) adapter. A non-limiting example of a wired communication interface device includes a universal serial bus (USB) adapter. Additionally, alternative communication interface embodiments including wired or wireless network interface adapters that provide a compatible communication interface between the electronic device 104 and the analyte sensor 140 may be used as well. Additionally, the wired or wireless network interface adapters in the communication interface 112 provide access to a network 150, such as a wide area network (WAN) or local area network (LAN), that enable the electronic device 104 to send and receive data with the online data service 160.

In the electronic device 104, the display device 116 provides visual outputs of a graphical user interface (GUI) to the PwD 102 and the input device 118 enable the PwD 102 to control the operation software applications, respectively. The display device 116 is, for example, a liquid crystal display (LCD), organic light emitting diode (oLED), MicroLED, or other suitable visual display device. The input device 118 includes any hardware interfaces that enable the user 102 to enter data or commands to control the functions of software, including the first DMA 122 and the second DMA 124, which the processor 108 executes during operation. Examples of input devices include input button, scroll wheel, keyboard, mouse, touchscreen, audio microphone input devices, and the like. In particular, in some embodiments a touchscreen input is physically integrated with the display device 116 to enable the user 102 to press graphical icons, enter gestures, and perform tactile interaction with other on-screen elements that the processor 108 generates with the display device 116.

In the electronic device 104, the memory 120 includes one or more volatile data storage devices such as random access memory (RAM) and non-volatile data storage devices such as solid state memory, magnetic, or optical media that store programmed instructions and data to control the operation of the electronic device 104. In particular, FIG. 1 depicts the memory 120 including the stored program instructions 122 of the first DMA 122, second DMA 124, and a set of time series blood glucose data 128. For illustrative purposes, in the examples provided herein the first DMA 122 is a DMA that provides a detailed analysis of the blood glucose data 128 including long-term trend data, such as trends of GMI levels over various time ranges, changes in the TIR over time, and predictions of future blood glucose trends. The second DMA 124 provides a simplified access to the blood glucose data 128 that are received from the analyte sensor 140, and optionally data from other data sources that are relevant to the PwD 102, to enable the PwD 102 to review his or her current diabetes and health status. Additionally, the second DMA 124 provides an input user interface for the PwD 102 to enter log book entry data 128 that the processor 108 stores in the memory 120, although both DMAs 122 and 124 may access the log entries. For example, the second DMA 124 provides a simplified view of current blood glucose levels from the analyte sensor 140, of a current glucose management indicator (GMI), a recent history of time in range (TIR), and a current blood glucose level to the PwD 102. More generally, the first DMA 122 provides analysis of long-term trends and disease management for the PwD 102, while the second DMA 124 provides data for short-term monitoring of blood glucose and other indicators for acute diabetes management and records log book information for diabetes management events.

During operation, both the first DMA 122 and second DMA 124 access the time series blood glucose data 128 that the electronic device 104 receives from the analyte sensor 140. In the illustrative embodiment of FIG. 1, the memory 120 stores a single copy of the time series blood glucose data in a database or other structured data format that associates each blood glucose measurement with a timestamp and optionally other medically relevant data for the PwD 102, such as data from a smart watch or other physiological monitoring device. FIG. 1 depicts a single instance of the time series blood glucose data 128 for illustrative purposes, but in some embodiments the memory 120 stores separate copies of the blood glucose data 128 for the first DMA 122 and the second DMA 124 due to security restrictions in some commercially available operating systems that prevent direct sharing of data between software applications, or for other implementation-specific reasons. Furthermore, in the system 100 the online data service 160 optionally stores additional time series blood glucose data 164 corresponding to the PwD 102. The online data service 160 is, for example, a commercially available networked data storage service, healthcare provider service, or other networked computing system that is coupled to the electronic device 104 via the network 150 to enable the electronic device 104 to store and retrieve time series blood glucose data and optionally other medically relevant data for the PwD 102. In one configuration, the time series blood glucose data 164 store a copy of the time series blood glucose data 128 to, for example, provide backup storage in case the electronic device 104 fails and to enable each DMA to retrieve a copy of the blood glucose data from the online data service 160 in electronic device configurations that prevent direct data sharing between applications. Additionally, in some embodiments the memory 120 in the electronic device 104 has a limited storage capacity for the time series blood glucose data 128, and typically only stores the most recent data over a predetermined time range (e.g. 24 hours or seven days of data). The online data service 160 optionally stores an archive of the time series blood glucose data 164 with a longer history for the PwD 102 over a span of multiple days, weeks, months, or optionally years. The first DMA 122 and the second DMA 124 access the archived time series blood glucose data 164 on an as-needed basis to provide trend data over time for diabetes management parameters and current-time displays of diabetes management parameters to the PwD 102.

In the system 100, the analyte sensor 140 is a device that generates physiological measurements of at least one chemical analyte in the body of the user 102. In most instances, the chemical analyte being measured is a level of blood glucose, which the analyte sensor 140 measures directly or indirectly. In other configurations, the analyte sensor 140 detects other analytes such as ketones. In any of these embodiments, the physiological measurements form the basis for the determination of diabetes management parameters either directly, such as direct use of blood glucose level measurements, or serve as a basis for the calculation of diabetes management parameters, such as GMI, TIR, and future blood glucose value prediction calculations. In one embodiment, the analyte sensor 140 is a commercially available continuous glucose monitor (CGM) device that is affixed to the body of the user 102. The CGM analyte sensor 140 measures blood glucose values indirectly via interstitial fluid in the body of the user 102. A CGM analyte sensor 140 generates blood glucose measurements at regular intervals such as every minute, every five minutes, or at another periodic rate that enables the generation of a time series of physiological measurements, such as a time series of blood glucose level measurements in the user 102.

In the system 100, the analyte sensor 140 includes a sensor controller 144, a sensor element 146, a communication interface 148, and a memory 152. The sensor controller 144 is a digital, analog, or a combined digital/analog control device that is operatively connected to the sensor element 146, communication interface 148, and the memory 152. In most embodiments, the sensor controller 144 executes firmware instructions stored in the memory 152 (not shown) during operation. The sensor element 146 includes any electrochemical or other sensing hardware that perform detection of the analyte in the body of the user 102 to generate each physiological measurement. The sensor controller 144 operates the sensor element 146 to generate measurements of an analyte, such as blood glucose, and stores one or more diabetes management parameters in the memory 152 as a set of time series blood glucose data 154. The communication interface 148 in the analyte sensor 140 is another communication interface device that is compatible with the corresponding communication interface 112 in the electronic device 104. During operation, the sensor controller 144 operates the communication interface 148 to transmit the stored time series blood glucose data 154 to the electronic device 104. The electronic device processor 108 stores the data as the time series blood glucose data 128 in the memory 120. In one operating mode, the sensor controller 144 transmits each physiological measurement datum to the electronic device 104 with minimal delay after generation of each measurement, while in other embodiments the memory 152 stores a set of multiple measurements in a time series and transmits batches of multiple physiological measurements to the electronic device 104 either periodically or in response to an on-demand request from the electronic device 104. Each blood glucose measurement in the time series blood glucose measurement data 154 includes data corresponding to the measurement of the analyte, such as a quantitative value for the measured blood glucose level in the user 102, a timestamp indicating when the measurement was generated, and any other metadata necessary to track the measured analyte in the user 102 over time.

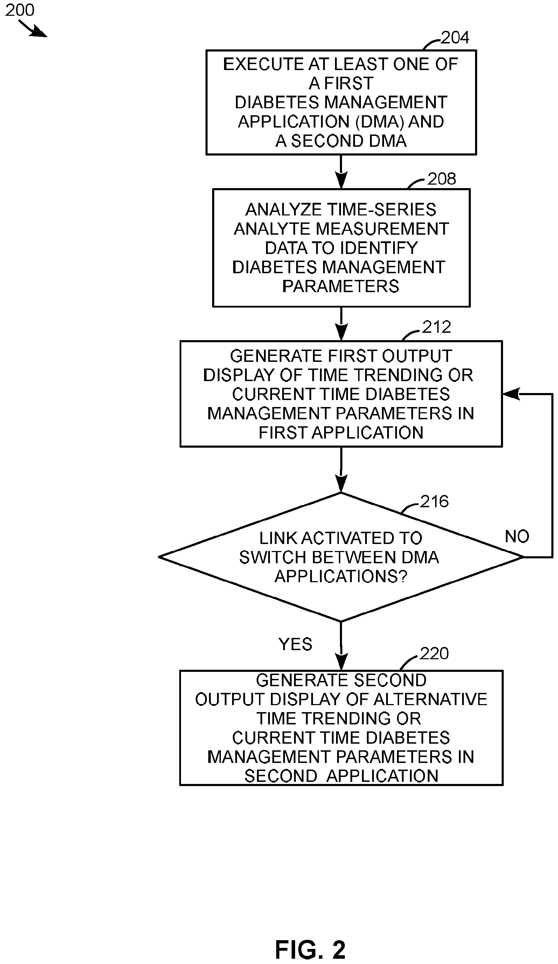

FIG. 2 is a block diagram of a process 200 for operation of an electronic device that executes multiple diabetes management applications that include complementary displays of current time and time trending diabetes management parameters. In the process 200, a software developer provides the stored instructions for at least one diabetes management application for storage in the memory and execution by a processor in an electronic device, such as the memory 120 and the processor 108 in the electronic device 104 of FIG. 1. In the description below a reference to a function or operation of process 200 refers to the operation of one or more processors to execute stored program instructions to perform the function or operation in conjunction with other components in a diabetes management system. The process 200 is described in conjunction with the system 100 of FIG. 1 for illustrative purposes.

The process 200 begins with execution of at least one of the first DMA and the second DMA (block 204). In the electronic device 104, the processor 108 reads and executes the stored instructions of either or both of the first DMA 122 and the second DMA 124. The DMAs may be executed in any order or be executed simultaneously in embodiments of the electronic device 104 that enable parallel execution of multiple software programs. Additionally, in some configurations, the processor 108 only executes one of the DMAs 122 or 124 initially and the processor 108 executes the other DMA at a later time in response to selection of a shortcut link in a user interface. In the embodiment of FIG. 1, the electronic device 104 generates a display of one of the DMAs 122 or 124 via the display device 116 even if both DMAs are executed concurrently, while the DMA 122 or 124 that is not displayed may be suspended or continue to perform operations in the background. The processor 108 can change the display between the DMAs 122 and 124 in response to input from the user 102 via the input device 118 or from programmed instructions in the DMAs 122 and 124.

The process 200 continues as the processor 108 executes either or both of the DMAs 122 and 124 to analyze the time series blood glucose data or other time series analyte data to identify one or more diabetes management parameters (block 208). Non-limiting examples of diabetes management parameters that the DMAs 122 and 124 are configured to identify include the glucose management index (GMI), time in range (TIR), and blood glucose level diabetes management parameters. In particular, the first DMA 122 utilizes algorithms that are otherwise known to the art to identify time trends in diabetes management parameters such as GMI, TIR, and predictions of future blood glucose levels for the PwD 102 based at least in part on the time series blood glucose data 128. The first DMA 122 optionally stores results of previously identified diabetes management parameters in the memory 120 or in the remote online data service 160 to enable the first DMA 122 to generate output displays of trends in the diabetes management parameters over time. The second DMA 124 also identifies one or more diabetes management parameters based at least in part on the time series blood glucose data 128, and identifies current time diabetes management parameter values to generate output displays of the current time diabetes management parameter data for the PwD 102.

In some configurations, one or both of the first diabetes management application 122 and the second diabetes management application 124 retrieves the analyte data, such as the time series blood glucose data 154, from the analyte sensor 140 using the communication interface 112 in addition to analyzing the analyte data to identify diabetes management parameters. In the embodiment of FIG. 1, either or both of the DMAs 122 and 124 include instructions to operate the processor 108 and the communication interface 112 to retrieve time series analyte data from an external sensor device, such as the time series blood glucose data 154 in the memory 152 of the analyte sensor 140 in the system 100. As described above, in some configurations the DMAs 122 and 124 processor 108 also retrieves copies of the time series blood glucose data or other time series analyte data from the online data service 160. In alternative configurations, a separate software service (not shown) retrieves the time series analyte data, such as the time series blood glucose data 154 in the memory 152 of the analyte sensor 140, using the communication interface 112, this enables the electronic device 104 to receive the time series blood glucose data 128 when neither the first DMA 122 nor second DMA 124 are activated.

The process 200 continues as either the first DMA 122 or the second DMA 124 generates an output display of the time trending or current time diabetes parameters in a first selected DMA (block 212). During operation, the first selected DMA is either the first DMA 122 or the second DMA 124 based either on a user input from the PwD 102 or optionally due to an diabetes management related event occurring that triggers the processor 108 to execute either the first DMA 122 or the second DMA 124 to generate a display via the display device 116. In different configurations, either the first DMA 122 or the second DMA 124 generates an initial output display and both of the DMAs are configured to enable efficient switching between user interface displays of the diabetes management parameters.

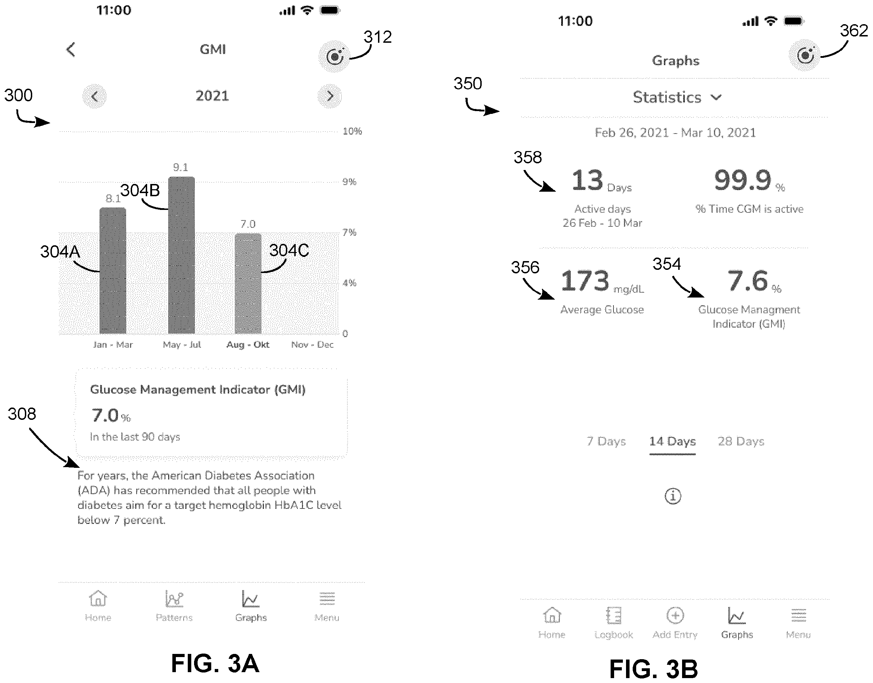

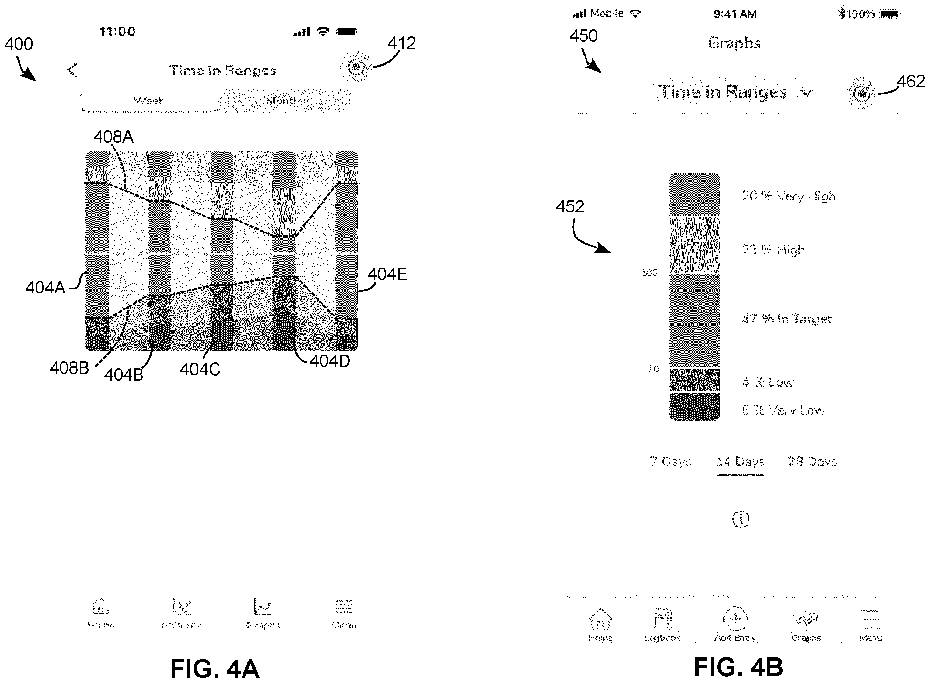

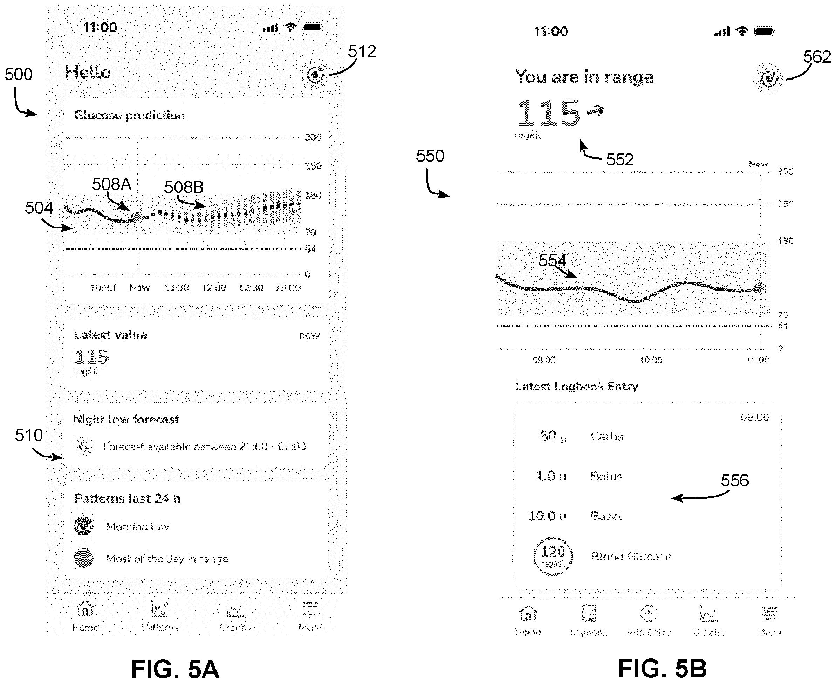

Using the first DMA 122 as a non-limiting example of the first selected DMA, FIGs. 3A, 4A, and 5A depict output displays of a the diabetes management parameters depicting a trend of diabetes management parameter levels over time. In the examples below, FIG. 3A depicts an output display 300 of GMI diabetes parameter data, FIG 4A depicts an output display 400 of TIR diabetes parameter data, and FIG. 5A depicts an output display 500 of predicted future blood glucose diabetes parameter data.

Referring to FIG. 3A, the output display 300 includes graphical indicators, such as bars 304A, 304B, and 304C in a bar chart display, with of an average GMI occurring in at least one prior time range and a second graphical indicator of an average GMI occurring in a present time range. In more detail, the bars 304A and 304B each depict the average GMI that was measured over prior three-month time ranges, and the bar 304C indicates the currently measured GMI over the most recent three month time range. The bar graph depicts the trend of changes in GMI over time for the PwD 102 to review GMI trends. The output display 300 optionally includes additional information 308 pertaining the GMI for the PwD 102. The output display 300 further includes a graphical indicator of a link 312 that enables the PwD 102 to switch from the display of GMI in the first DMA 122 to a related alternative simplified display of the current time GMI in the second DMA 124 (see FIG. 3B). The graphical indicator 312 is depicted as a set of circles indicating the option to switch the output display in FIG. 3A, but alternative graphical indicator links may include different graphics, text, animations, or other indicators to enable the PwD 102 to switch between displays in the DMAs 122 and 124.

Referring to FIG. 4A, the output display 400 includes graphical indicators, such as bars 404A - 404E, in a bar chart display, with of TIR occurring in a series of prior time ranges leading to the current time TIR. To display relative TIR, the output display 400 subdivides each bar 404A - 404E into three colored segments indicating hyperglycemia (orange/yellow, top of bar), in-range glycemic index values (green, middle of bar), and hypoglycemia (light/dark red, bottom of bar). The relative proportion of each colored region in each of the bars 404A - 404E indicates the relative amount of time that the PwD 102 spends either in-range or out of range in a hyperglycemic or hypoglycemic state. Each bar 404A - 404E depicts a predetermined time range, such as a day, week, etc. that the first DMA summarizes to depict the TIR performance during the predetermined time range. Additionally, the output display 400 includes trend lines 408A/408B that indicates changes in TIR over time between the different time ranges. In the example of FIG. 4, the final bar 404E indicates the current TIR for the PwD over the most recent day or other predetermined time frame. The output display 400 further includes a graphical indicator of a link 412 that enables the PwD 102 to switch from the display of TIR in the first DMA 122 to a related alternative simplified display of the TIR in the second DMA 124 (see FIG. 4B).

Referring to FIG. 5A, the output display 500 includes graphical indicators, such as a linear graph 504, of a prediction of future analyte values. In more detail, a graph segment 508A depicts a linear graph of measured analyte values, such as measured blood glucose values in the time series blood glucose data 128, for a predetermined time range up to the current time, and second graph segment 508B includes a trend line of future predicted blood glucose values over another predetermined time range, such as a two hour time range depicted in FIG. 5A. The graph segment 508B depicts the predicted blood glucose values, and further includes error bars indicating potential variations from the precise predicted values. While not expressly depicted in the example of FIG. 5A, in some embodiments the linear graph 504 also includes color coding to indicate if a measured or predicted blood glucose value in either of the graph segments 508A or 508B corresponds to a hyperglycemic condition (yellow/orange), an in-range condition (green) or a hypoglycemic condition (light or dark red). The graphical display 500 further includes pattern data 510 that informs the PwD 102 of general patterns in the daily blood glucose values, such as an indication of a prediction for low blood glucose levels experience overnight and patterns of observed low blood glucose levels over a prior 24 hour window including indicia of low blood glucose levels in the morning and that the PwD 102 spent most of the 24 hour window in-range. The output display 500 further includes a graphical indicator of a link 512 that enables the PwD 102 to switch from the display of blood glucose trend data in the first DMA 122 to a related alternative simplified display of the current blood glucose level in the second DMA 124 (see FIG. 5B).

Using the second DMA 124 as a non-limiting example of another first selected DMA, FIGs. 3B, 4B, and 5B depict output displays of a the diabetes management parameters depicting current-time values of diabetes management parameter levels in a simplified user interface display. In the examples below, FIG. 3B depicts an output display 350 of GMI diabetes parameter data, FIG 4B depicts an output display 405 of TIR diabetes parameter data, and FIG. 5B depicts an output display 550 of blood glucose diabetes parameter data.

Referring to FIG. 3B, the second DMA 124 generates an output display 350 that indicates the GMI 354 as a single value (e.g. a percentage value of 7.6% in the illustrative example of FIG. 3B). This value is calculated over a time range, such as a 14 day range in FIG. 3B, although the second DMA 124 may use shorter or longer time ranges. While the value of the GMI 354 is calculated using data that extend over a prior time range, the output display 350 displays a current-time GMI because the GMI 354 is the most up-to-date calculated value for the PwD 102 at the time that the processor 108 generates the output display 350. The output display 350 further includes other optional data such as an average glucose level display 356 for the PwD 102 over the time rang and an operational data display 358 for the analyte sensor 140 such as the number of active days and the active time percentage for the CGM during the active days. The output display 350 also includes the graphical indicator of a link 362 that enables the PwD 102 to switch to the display 300 in the first DMA 122.

Referring to FIG. 4B, an output display 450 includes graphical indicators, such as a bar graph 452, that depicts the current-time TIR for the PwD 102 over a single time range, such as a 14 day range as depicted in the output display 450 although longer or shorter time ranges may be selected. The linear graph 452 depicts the proportion of time that the PwD 102 has spent in-range using a green colored bar with a size that is proportional to the total time spent in-range (e.g. 47% in FIG. 4B). The graph 452 also includes color coding to indicate the proportion of time spent in hyperglycemia (orange/yellow) or hypoglycemia (light/dark red). The output display 450 further includes a graphical indicator of a link 462 that enables the PwD 102 to switch from the output display 450 to the related TIR trend graphs in the output display 400 for the first DMA 122 that is depicted in FIG. 4A.

Referring to FIG. 5B, an output display 550 includes a simplified display 552 of the current-time blood glucose level value as a numeric unit (e.g. mg/dL or mmol/L) and a simplified indicator if the blood glucose level is in-range for the PwD 102. The output display 550 further includes a historic graph 554 showing a short time range (e.g. 2 hours) of prior measured blood glucose levels. The simplified display also depicts the most recent log book data entry 556, which includes data corresponding to the blood glucose level, carbohydrate intake for a meal, and bolus size for an insulin dose in the example of FIG. 5B. The output display 550 also includes the graphical indicator of a link 562 that enables the PwD 102 to switch to the display 500 in the first DMA 122.

Referring again to FIG. 2, the process 200 continues as the PwD 102 optionally selects the link in the output display of one of the two DMAs 122 or 124 and the processor 108 activates the corresponding output display in the other of the two DMAs 122 or 124 (block 216). For example, if the processor 108 executes the first DMA 122 to generate the GMI output display 300, then the processor 108 activates the second DMA 124 to generate the GMI output display 350 in response to receiving a selection of the graphical indicator of the link 312 (block 220). In reverse, if the processor 108 executes the second DMA 124 to generate the output display 350, then the processor 108 activates the first DMA 122 to generate the output display 300 in response to receiving a selection of the graphical indicator of the link 362 via the input device 118 (block 220). Similarly, the processor 108 can activate the appropriate DMA 122 or 124 to switch between the output displays 400 and 450 for TIR diabetes management parameters as depicted in FIGs. 4A and 4B and to switch between the output displays 500 and 550 for blood glucose diabetes management parameters as depicted in FIGs. 5A and 5B. To activate the DMA that is not currently displayed, the processor 108 either switches the graphical output via the display device 116 to the alternate DMA if the alternate DMA is already being executed in the background, or the processor 108 executes the alternate DMA and the alternate DMA performs the functions described above with reference to block 208 to analyze the analyte data and generate the appropriate output display. During the process 200, the processor 108 may alternate between the related output displays of the diabetes parameter data in the DMAs 122 and 124 multiple times in response to selections of the links. The process 200 continues to generate the originally selected output display if the link is not activated as described above with reference to the processing of block 212.

The embodiments described herein provide several improvements to the operation of electronic devices that operate diabetes management applications and to the PwDs who rely on the diabetes management applications during ongoing treatment for diabetes. Non-limiting examples of these improvements include enabling the display of complex diabetes management parameter data such as the time series data of GMI, TIR, and predicted blood glucose data in juxtaposition with simplified versions of the same GMI, TIR, and blood glucose parameters using different DMA software applications. This enables the PwD to review time series analysis of the diabetes management parameters for long-term diabetes management while also providing reduced cognitive load for short-term diabetes management decisions, such as taking short-term action to manage blood glucose levels. Additionally, the first DMA 122 and the second DMA 124 are configured to interoperate with links to related diabetes management parameter output displays that reduces the need for the PwD to manually execute both applications and navigate through the full user interface of both applications to review similar types of diabetes management parameter data. In some configurations, the interoperability also enables DMAs from different manufacturers to interoperate without requiring a single DMA application. Additionally, the embodiments described herein improve the efficiency of operation for the electronic device 104 in at least two ways. First, since two different applications present diabetes parameter data in two different ways the electronic device 104 can save memory and processing time by only generating a display of one set of diabetes parameter data at a time in response to the selection from the PwD 102. Second, the links that enable efficient switching between the time series and current time output displays for each DMA not only simplify the operation for the PwD 102, but enable more efficient operation of the processor 108 in the electronic device 104 because the processor 108 can omit unnecessary operations in the DMAs and instead generate output displays of the relevant selected diabetes parameter data.

This disclosure is described in connection with what are considered to be the most practical and preferred embodiments. However, these embodiments are presented by way of illustration and are not intended to be limited to the disclosed embodiments. Accordingly, one of skill in the art will realize that this disclosure encompasses all modifications and alternative arrangements within the spirit and scope of the disclosure and as set forth in the following claims.

## Claims

1. A method for generating output displays of diabetes management parameters comprising:
providing stored program instructions of a first diabetes management application, the first diabetes management application being configured to be stored in a memory of an electronic device and, upon execution by a processor in the electronic device, the first diabetes management application being configured to:
generate a first output display of a plurality of diabetes management parameters based at least in part on a time series of analyte data stored in the memory, the plurality of diabetes management parameters depicting a trend of diabetes management parameter levels over time;
generate a graphical indicator of a link to a second diabetes management application stored in the memory of the electronic device in the first output display; and
activate the second diabetes management application stored in the memory in response to an input selection of the link, wherein the second diabetes management application is configured to be executed by the processor to generate a second output display of a current-time diabetes management parameter based at least in part on the time series of the analyte data stored in the memory.

2. The method of claim 1, wherein the plurality of diabetes management parameters are glucose management indicators (GMIs) and the current-time diabetes management parameter is a current-time GMI.

3. The method of claim 2, wherein upon execution by the processor, the first diabetes management application is further configured to:
generate the first output display including a first graphical indicator of an average GMI occurring in a prior time range and a second graphical indicator of an average GMI occurring in a present time range.

4. The method according to any one of the preceding claims, wherein the plurality of diabetes management parameters are Time in Range (TIR) parameters and the current-time diabetes management parameter is a current-time TIR parameter.

5. The method of claim 4, wherein upon execution by the processor, the first diabetes management application is further configured to:
generate the first output display including a series of at least two bars and a trend line, wherein each bar includes color indications of TIR during a time period corresponding to the bar and the trend line indicates changes in TIR over time between the at least two bars.

6. The method according to any one of the preceding claims, wherein the plurality of diabetes management parameters are a trend of predicted analyte levels and the current-time diabetes management parameter is a current-time analyte level.

7. The method of claim 6, wherein upon execution by the processor, the first diabetes management application is further configured to:
generate the first output display including a linear graph of a prediction of future analyte values.

8. The method according to any one of the preceding claims, further comprising:
providing the stored program instructions of the second diabetes management application, the second diabetes management application being configured to be stored in the memory of the electronic device and to be executed by the processor in the electronic device.

9. The method according to any one of the preceding claims, wherein upon execution by the processor, the first diabetes management application is further configured to:
receive the time series of analyte measurement data from an analyte sensor, the time series of analyte data being accessible to the first diabetes management application and the second diabetes management application.

10. The method according to any one of the preceding claims, wherein the time series of analyte measurement data is a time series of blood glucose measurement data.

11. The method according to any one of the preceding claims, wherein the method is computer implemented.

12. An electronic device configured to generate diabetes management parameter user interfaces comprising:
a memory configured to store:
stored program instructions of a first diabetes management application; and
stored program instructions of a second diabetes management application;
a display device; and
a processor operatively connected to the memory and the display device, the processor configured to execute the first diabetes management application and the second diabetes management application to:
generate a first output display of a plurality of diabetes management parameters based at least in part on a time series of analyte data stored in the memory, the plurality of diabetes management parameters depicting a trend of diabetes management parameter levels over time;
generate a graphical indicator of a link to a second diabetes management application stored in the memory of the electronic device; and
activate the second diabetes management application stored in the memory in response to an input selection of the link to generate a second output display of a current-time diabetes management parameter based at least in part on the time series of the analyte data stored in the memory.

13. The electronic device of claim 12, wherein the plurality of diabetes management parameters are glucose management indicators (GMIs) and the current-time diabetes management parameter is a current-time GMI.

14. The electronic device of claim 13, wherein the processor is further configured to:
execute the first diabetes management application to generate the first output display including a first graphical indicator of an average GMI occurring in a prior time range and a second graphical indicator of an average GMI occurring in a present time range.

15. The electronic device according to any one of the preceding claims referring to an electronic device, wherein the plurality of diabetes management parameters are Time in Range (TIR) parameters and the current-time diabetes management parameter is a current-time TIR parameter.

16. The electronic device of claim 15, wherein the processor is further configured to:
execute the first diabetes management application to generate the first output display including a series of at least two bars and a trend line, wherein each bar includes color indications of TIR during a time period corresponding to the bar and the trend line indicates changes in TIR over time between the at least two bars.

17. The electronic device according to any one of the preceding claims referring to an electronic device, wherein the plurality of diabetes management parameters are a trend of predicted analyte levels and the current-time diabetes management parameter is a current-time analyte level.

18. The electronic device of claim 17, wherein the processor is further configured to:
execute the first diabetes management application to generate the first output display including a linear graph of a prediction of future analyte values.

19. The electronic device of according to any one of the preceding claims referring to an electronic device, wherein the processor is further configured to: receive the time series of analyte measurement data from an analyte sensor using a communication interface, the time series of analyte data being accessible to the first diabetes management application and the second diabetes management application.

20. The electronic device of according to any one of the preceding claims referring to an electronic device, wherein the time series of analyte measurement data is a time series of blood glucose measurement data.

21. A computer program comprising instructions which, when the program is executed by the electronic device according to any one of the preceding claims referring to an electronic device, cause the electronic device to perform the method according to any one of the preceding claims referring to a method.

22. A computer-readable storage medium comprising instructions which, when the instructions are executed by the electronic device according to any one of the preceding claims referring to an electronic device, cause the electronic device to perform the method according to any one of the preceding claims referring to a method.

23. A non-transient computer-readable medium including instructions that, when executed by one or more processors, cause the one or more processors to perform the method according to any one of the preceding claims referring to a method.
